# EUROPEAN PATENT APPLICATION

(11) **EP 4 242 291 A1**
(43) Date of publication of application: **13.09.2023**
(21) Application number: 20960265.5
(22) Date of filing: 04.11.2020
(51) Int. Cl.: C12M 1/34, G01N 21/64

(54) **TEST SYSTEM**

(71) Applicant: MGI Tech Co., Ltd., Shenzhen, Guangdong 518083 (CN)
(72) Inventor: HUANG, Yi, Shenzhen, Guangdong 518083 (CN); JIANG, Heming, Shenzhen, Guangdong 518083 (CN); WEI, Jie, Shenzhen, Guangdong 518083 (CN); LI, Songlin, Shenzhen, Guangdong 518083 (CN)
(74) Representative: Zaboliene, Reda
(86) International application number: PCT/CN2020/126532
(87) International publication number: WO 2022/094803

(57) **Abstract**

A test system (10), comprising: a carrier disc (40) configured to carry a target sample (20); a light source module (30) configured for emitting at least two types of reference light (L1, L2) with different wavelengths; a light guiding module (32) located on an emitting path of the reference light (L1, L2) and configured for guiding the reference light (L1, L2) to the target sample (20) and for receiving and guiding at least two types of detection light (L3, L4, L5) with different wavelengths, the detection light (L3, L4, L5) being generated by the target sample (20) according to the reference light (L1, L2); and a detection module (33) configured for receiving the detection light (L3, L4, L5) and obtaining biochemical information of the target sample (20) according to the detection light (L3, L4, L5).

## Description

### FIELD

The subject matter relates to the technical field of biochemical information detection, in particular to a test system.

### BACKGROUND

The rapid development of genetic testing technology has become the main force driving the rapid advancement of the biological industry.

A gene sequencer is a core equipment in the gene testing industry. The basis of the gene sequencer is to extract the base signal of biological materials, and the extraction of the base signal depends on the high-throughput fluorescent light test system. At present, most of imaging systems of gene sequencers use traditional fluorescent microscope lighting and imaging systems.

The above gene sequencer can achieve a high detection rate, but the cost is high, the equipment is large, and the installation and adjustment are extremely difficult. How to ensure a higher detection rate and luminous flux on the basis of effectively controlling the cost and volume of the equipment is a problem urgently to be solved in the art.

### SUMMARY

The present disclosure provides a test system, which includes:
a carrier disc configured to carry a target sample;
a light source module configured for emitting at least two types of reference light with different wavelengths;
a light guiding module located on an emitting path of the reference light and configured for guiding the reference light to the target sample and for receiving and guiding at least two types of detection light with different wavelengths, the detection light being generated by the target sample according to the reference light; and
a detection module configured for receiving the detection light and obtaining biochemical information of the target sample according to the detection light.

Preferably, the light source module includes a first laser source and a second laser source, the first laser source is configured to emit a first reference light, and the second laser source is configured to emit a second reference light;
the detection light includes a first detection light and a second detection light;
the detection module includes a first detector for receiving the first detection light and a second detector for receiving the second detection light, the first detector and the second detector are located in different optical paths, the detection module is configured to obtain the biochemical information of the target sample according to the first detection light and the second detection light.

Preferably, the first detector and the second detector are photodiodes.

Preferably, the detection light further includes part of the first reference light, and the test system further includes a focusing and tracking module;
the focusing and tracking module is configured to receive the first reference light in the detection light, and to obtain a focus signal and a tracking signal according to the first reference light in the detection light, and the focus signal and the tracking signal are configured to adjust positions where the first reference light and the second reference light are incident on the carrier disc.

Preferably, the light guiding module includes a first light splitting element, the first light splitting element is configured for guiding the reference light emitted by the light source module to the carrier disc, and for guiding the detection light to the detection module.

Preferably, the light guiding module further includes a second light splitting element, the second light splitting element is configured to guide the first detection light and the second detection light to the detection module, and to guide the first reference light in the detection light to the focusing and tracking module.

Preferably, the light guiding module further includes a third light splitting element, the third light splitting element is configured to guide the first detection light to the first detector, and to guide the second detection light to the second detector.

Preferably, the target sample is nucleic acid; the biological information is base sequence information of the target sample.

Preferably, the target sample carries a fluorescent substance; the reference light is laser light, and the detection light is fluorescent light.

Preferably, the carrier disc is made of glass.

The above test system replaces the traditional biochip with the carrier disc, which is beneficial to reduce the cost of the test system and also helps to reduce the volume of the test system.

### BRIEF DESCRIPTION OF THE DRAWINGS

FIG. 1 is a schematic structural diagram of a test system and target sample according to an embodiment of the present disclosure.
FIG. 2 is another schematic structural diagram of a test system and target sample according to the embodiment of the present disclosure.
FIG. 3 is still another schematic structural diagram of a test system and target sample according to the embodiment of the present disclosure.
FIG. 4 is a schematic structural diagram of an objective lens of a test system according to the embodiment of the present disclosure.
FIG. 5 is a schematic diagram of an optical path of a focusing and tracking module of the test system according to the embodiment of the present disclosure.
FIG. 6A is a schematic plan view of a light spot on a near-focus plane.
FIG. 6B is a schematic plan view of the light spot on a positive focus plane.
FIG. 6C is a schematic plan view of the light spot on a far focal plane.
FIG. 7 is a schematic plan view of a four-quadrant detector.
FIG. 8 is a schematic diagram showing the light intensity of the light received by the four-quadrant detector.

### Symbol description of main components:

Test system 10; target sample 20; detection device 30; light source module 31; first laser source 311; second laser source 312; first lens 313; second lens 314; light guiding module 32; light combining element 321; first light splitting element 322; objective lens 323; first lens unit 3231; second lens unit 3232; third lens unit 3233; second light splitting element 324; third light splitting element 325; detection module 33; first filter 331; first detector 332; second filter 333; second detector 334; focusing and tracking module 35; lens 351; astigmatic lens 352; four-quadrant detector 353; carrier disc 40; first reference light L1; second reference light L2; first detection light L3; second detection light L4; third detection light L5.

Implementations of the disclosure will now be described, by way of embodiments only, with reference to the above drawings.

### DETAILED DESCRIPTION

In order to more clearly understand the objects, features and advantages of the present disclosure, a further detailed description on the present disclosure with reference to embodiments of the present invention taken in conjunction with the accompanying drawings. It is noted that the embodiments and features in the embodiments of the present application may be combined with each other without conflict.

Much specific detail of the present disclosure is described as below to sufficiently understand the present disclosure. The described embodiments are only some embodiments of the present disclosure, rather than all the embodiments. The disclosure is illustrative only, and changes may be made in the detail within the principles of the present disclosure. It will, thus, be appreciated that the embodiments may be modified within the scope of the claims.

Unless otherwise defined, all technical and scientific terms used herein have the same meaning as commonly understood by one of ordinary skill in the technical field of the present disclosure. The terms used herein in the description of the present disclosure are for the purpose of describing specific embodiments only, and are not intended to limit the present disclosure.

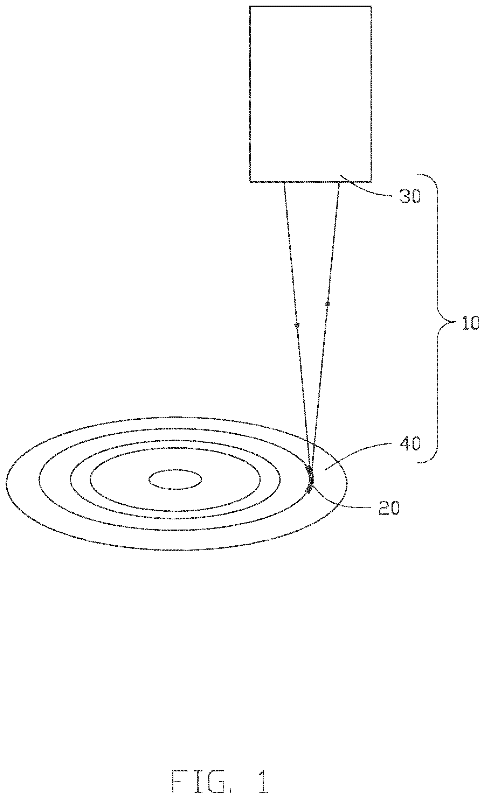

Referring to FIG. 1, a test system 10 is configured to detect biochemical information of a target sample 20. The target sample 20 can be a nucleic acid sample (DNA or RNA), protein, or cell, etc. In this embodiment, the target sample 20 is a nucleic acid sample, and the biochemical information may be base sequence information of the target sample 20.

Referring to FIG. 1, the test system 10 includes a detection device 30 and a carrier disc 40. The carrier disc 40 is configured to carry the target sample 20. During a working process of the test system 10, the detection device 30 emits reference light onto the carrier disc 40. In the working process, the carrier disc 30 is rotated so that the reference light is focused to different positions of the carrier disc 40, so as to focus the reference light to different positions of the target sample 20, thereby detecting the target sample 20. The carrier disc 40 can be fixed on a mechanical platform, and the mechanical platform is driven to rotate by a driving device (such as a motor) so as to drive the carrier disc 40 to rotate. When used for nucleic acid detection, the carrier disc 40 may be called a sequencing chip.

The reference light mainly includes light for focusing, light for tracking, and light for exciting fluorescent substances. The reference light is incident on the target sample 20, the target sample 20 generates detection light, and the detection light is received by the detection device 30, and the detection device 30 performs calculations based on the detection light to obtain the biochemical information of the target sample 20.

The power of the reference light is relatively large (on the order of hundreds of milliwatts), which may result in thermal issue. The detection light has a wide spectrum, which may result in chromatic aberration. The test system 10 may include one or more lens elements for guiding the light. In this embodiment, the above one or more lens elements are made of glass, which is conducive to heat dissipation of the test system 10 and is conducive to improving the chromatic aberration. In other embodiments, the above one or more lens elements can also be made of plastic; or some of the lens elements are made of glass and others are made of plastic.

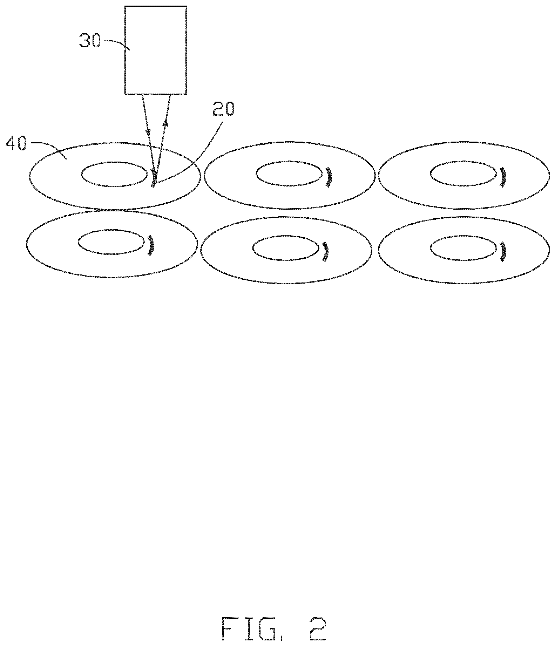

Referring to FIG. 2, in another embodiment, the test system 10 includes a plurality of carrier discs 40, and each carrier disc 40 carries a target sample 20. During the working process of the test system 10, the reference light is time-divisionally focused on different carrier discs 40. Each carrier disc 40 focuses when receiving the reference light, and rotates after focusing so that the reference light is focused and transmitted to different positions of the target sample 20, so as to detect the target sample 20.

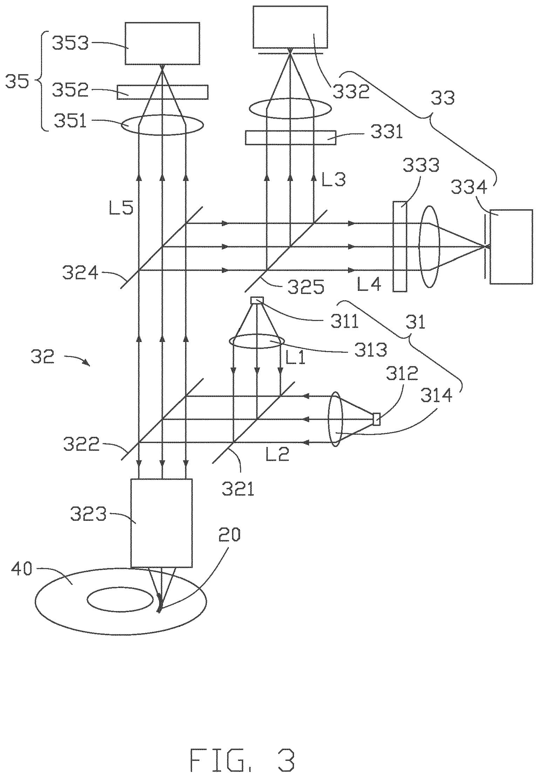

Referring to FIG. 3, the detection device 30 includes a light source module 31, a light guiding module 32 and a detection module 33.

The light source module 31 is configured to emit the reference light. The reference light includes at least two types of laser light with different wavelengths. The number of types of laser wavelengths in the reference light is determined according to the type of fluorescent substance configured to mark the target sample 20. In this embodiment, the target sample 20 carries two types of fluorescent substances, and the two types of fluorescent substances mark four bases by permutation and combination. For example, a first type of base carries a first type of fluorescent substance, a second type of base carries a second type of fluorescent substance, a third base carries the first and second types of fluorescent substances at the same time, and the fourth base does not carry a fluorescent substance. So that the light source module 31 includes a first laser source 311 for emitting the first reference light L1 and a second laser source 312 for emitting the second reference light L2. In other embodiments, there may be more than two types of fluorescent substances marking the target sample 20 , and the reference light may include more than two types of laser light with different wavelengths.

In this embodiment, the first reference light L1 is red laser light, and the second reference light L2 is green laser light. Both the first laser source 311 and the second laser source 312 are laser diodes. During a working process of the light source module 31, the first laser source 311 and the second laser source 312 respectively emit the first reference light L1 and the second reference light L2 simultaneously.

The light source module 31 further includes a first lens 313 and a second lens 314. The first lens 313 is located on an emitting path of the first reference light L1 and is configured for expanding and collimating the first reference light L1. The second lens 314 is located on an emitting path of the second reference light L2 and is configured for expanding and collimating the second reference light L2.

The light guiding module 32 is located on emitting paths of the first reference light L1 and the second reference light L2, and is configured to guide the first reference light L1 and the second reference light L2 to the carrier disc 40 (the target sample 20). In this embodiment, the paths of light emitted by the first laser source 311 and the second laser source 312 are different. The light guiding module 32 includes a light combining element 321. The light combining element 321 combines the first reference light L1 and the second reference light L2, so that the first reference light L1 and the second reference light L2 is transmitted along a common optical path. The light combining element 321 is a dichroic mirror for reflecting the first reference light L1 and transmitting the second reference light L2. In other embodiments, the light combining element 321 can also be configured to transmit the first reference light L1 and reflect the second reference light L2.

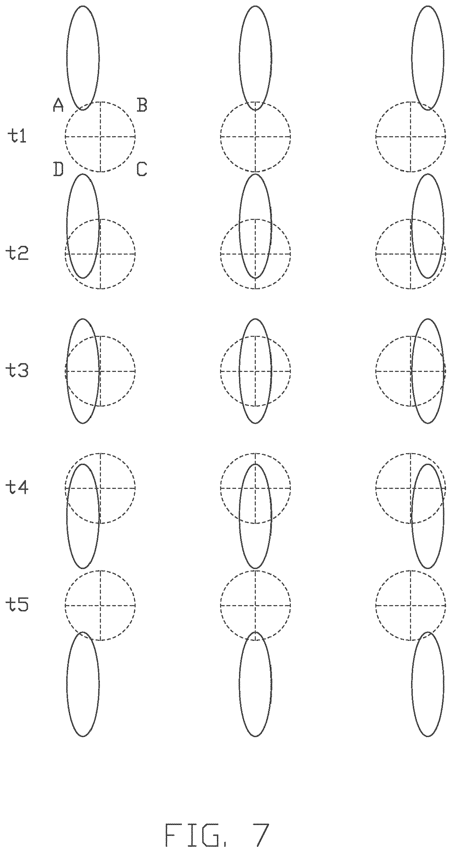

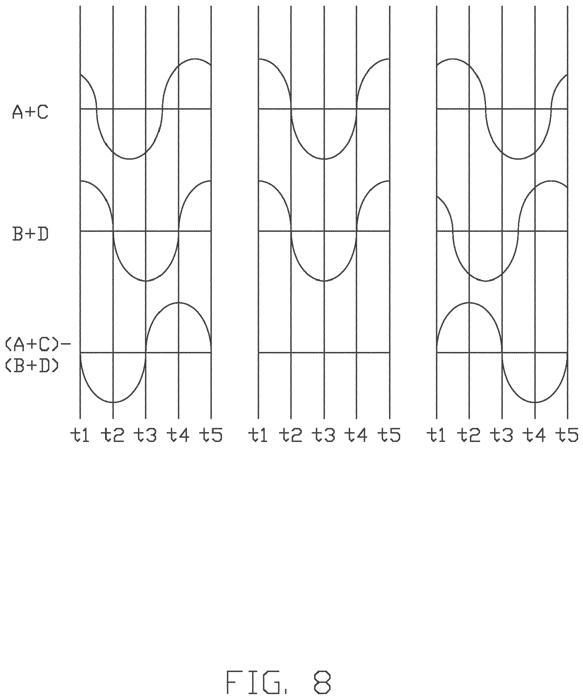

The light guiding module 32 further includes a first light splitting element 322 and an objective lens 323 . The first light splitting element 322 is located on the optical paths of the first reference light L1 and the second reference light L2 and is configured to guide the combined first reference light L1 and second reference light L2 to the objective lens 323. The objective lens 323 is configured to focus the combined first reference light L1 and second reference light L2 onto the carrier disc 40 (the target sample 20). In this embodiment, the first light splitting element 322 is a dichroic mirror, which is configured to reflect the first reference light L1 and the second reference light L2 to the objective lens 323, and is configured to transmit part of useful light, such as the first/second detection light mentioned below, the light returned from the carrier disc 40 and configured for focusing (the first reference light L1 incident to the four-quadrant detector), and the light configured for tracking (light in the tracking process shown in FIG. 7 and FIG. 8).

The target sample 20 on the carrier disc 40 is marked by two different fluorescent substances, one of which can be excited by the first reference light L1 to generate the first detection light L3, and the other fluorescent substance can be excited by the second reference light L2 to generate the second detection light L4. The first detection light L3 and the second detection light L4 are fluorescent light in different wavelength ranges. The first detection light L3 and the second detection light L4 are sequentially received by the objective lens 323 and the first light splitting element 322. In other embodiments, according to the types of fluorescent substances, the light source module 31 can emit more than two types of reference light, and the target sample can generate more than two types of detection light.

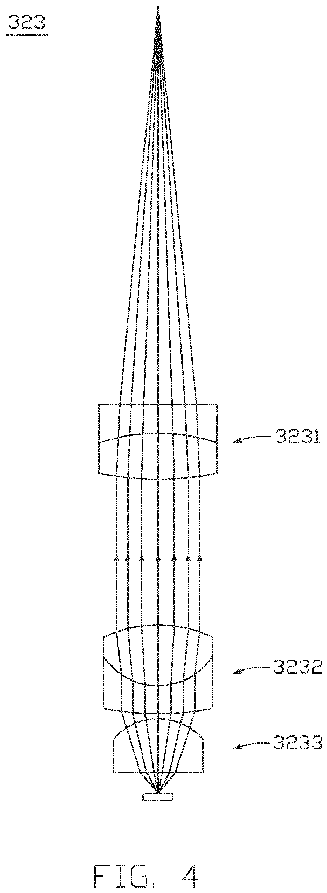

In this embodiment, the wavelength range of the first detection light L3 and the second detection light L4 is 550 nm to 740 nm. The spectrum of the first detection light L3 and the second detection light L4 is wide. In this embodiment, controlling the first detection light L3 and the second detection light L4 to form diffraction-limited light spots after passing through the objective lens 323 is beneficial to correct the chromatic aberration caused by the wide spectrum. Referring to FIG. 4, the objective lens 323 includes three lens units, which are lens unit 3231, 3232, and 3233 respectively. The first lens unit 3231 includes a convex lens and a concave lens that are bonded to each other, and the second lens unit 3232 also includes a convex lens and a concave lens bonded to each other. A numerical aperture of the objective lens 323 is set to 0.6, the wave aberration is less than 0.07λ, and the objective lens 323 is suitable for 0.25 mm cover glasses.

Referring to FIG. 3, the light guiding module 32 further includes a second light splitting element 324 and a third light splitting element 325. The first light splitting element 322, the second light splitting element 324, and the third light splitting element 325 are configured to coordinately guide the first detection light L3 and the second detection light L4 to the detection module 33. In this embodiment, both the second light splitting element 324 and the third light splitting element 325 are dichroic mirrors. The first light splitting element 322 is configured to transmit the first detection light L3 and the second detection light L4 to the second light splitting element 324, and the second light splitting element 324 is configured to reflect the first detection light L3 and the second detection light L4 to the third light splitting element 325. The third light splitting element 325 is configured to reflect the first detection light L3 and transmit the second detection light L4.

In other embodiments, the optical components of the light guiding module 32 (such as the aforementioned light combining elements, light splitting elements, lenses, etc.) can be different in type, quantity, combination, arrangement, etc. There are no specific limitations on the type, quantity, combination, and arrangement of the optical components, as long as the light can be transmitted along the aforementioned path.

The detection module 33 includes a first detection assembly for receiving the first detection light L3 and a second detection assembly for receiving the second detection light L4. The first detection assembly and the second detection assembly are located on different optical paths.

The first detection assembly includes a first filter 331 and a first detector 332. The first filter 331 is configured to filter out stray light generated in the test system 10 (such as the part of the laser light emitted by the laser source and reflected by some components to the first detection assembly), and to transmit the first detection light L3 to the second detector 332.

In this embodiment, the first detector 332 is an avalanche photodiode (APD). In other embodiments, the first detector 332 is a single photon avalanche diode (SPAD), or a photomultiplier tube (PMT). In another embodiment, the first detector 332 is a camera. When the first detector 332 adopts APD, SPAD or PMT, it can directly output corresponding electrical signal through photoelectric conversion of received optical signal. Compared with using a camera to take pictures, complex calculations in the image analysis process are omitted, which is conducive to speeding up data processing, thereby improving the detection speed of the test system 10.

The first detector 332 is configured to receive the first detection light L3, to generate a corresponding electrical signal according to the optical signal (first detection light L3), and to calculate and acquire the biochemical information of the target sample 20 according to the electrical signal and the pre-stored calculation method.

The second detection assembly includes a second filter 333 and a second detector 334. The types, arrangement order, and working principles of the components in the first detection assembly and the second detection assembly are basically the same, and will not be repeated here.

The detection device 30 further includes a control module (not shown) electrically connected to the light source module 31 and the detection module 33.

In this embodiment, the control module (not shown) is electrically connected to the first laser source 311, the second laser source 312, the first detector 332, and the second detector 334, and is configured to control the first laser source 311 and the second laser source 312 to emit the first reference light L1 and the second reference light L2 synchronously, and is configured to control the first detector 332 and the second detector 334 to calculate the biochemical information of target sample 20 according to the first detection light L3 and the second detection light L4 respectively.

When the test system 10 is working, the carrier disc 40 is in a rotating state, and the position on the carrier disc 40 where the objective lens 323 focuses the first reference light L1 and the second reference light L2 may deviate. The test system 10 further includes a focusing and tracking module 35. When the first reference light L1 and the second reference light L2 are incident on the carrier disc 40 , the carrier disc 40 also reflects part of the first reference light L1. That is, the detection light emitted from the carrier disc 40 to the objective lens 323 includes not only the first detection light L3 and the second detection light L4, but also part of reflected first reference light L1. The first reference light (that is, the reference light in the detection light) emitted from the carrier disc 40 to the objective lens 323 is defined as a third detection light L5. The focusing and tracking module 35 is configured to improve the above-mentioned focus position deviation problem according to the third detection light L5.

Referring to FIG. 3, the focusing and tracking module 35 includes a lens 351 , an astigmatic lens 352, and a four-quadrant detector 353 . In this embodiment, the four-quadrant detector 353 includes four photodiodes.

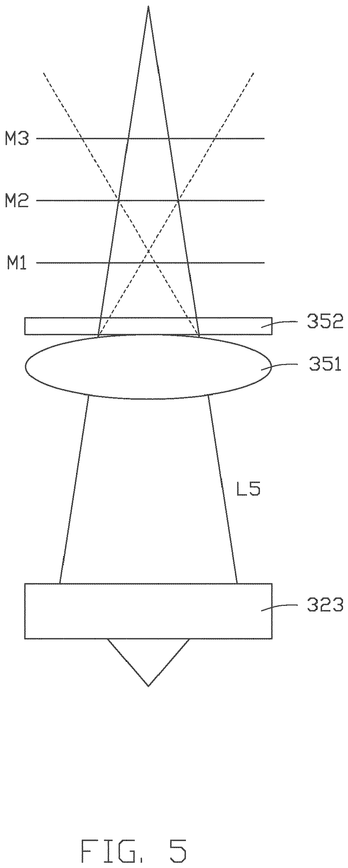

Referring to FIG. 3 and FIG. 5, the third detection light L5 is guided by the objective lens 323 to the lens 351 and the astigmatism lens 352 in turn, and produces astigmatism in the four-quadrant detector 353. Axial asymmetry appears in the astigmatic beam near a focal point (M2), and horizontal or vertical astigmatic lines appear on both sides of the focal point (M1 and M3).

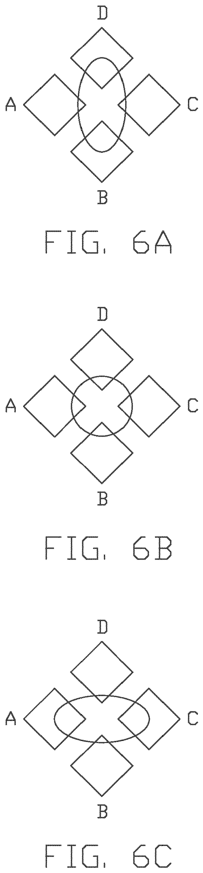

Referring to FIG. 6A, when the objective lens 323 is on a positive focal plane, the four-quadrant detector 353 receives a circular light spot, and the light intensities in four quadrants (A, B, C, D) are the same. Referring to FIG. 6B, when the objective lens 323 is on a far focal plane, the four-quadrant detector 353 receives an elliptical light spot. Referring to FIG. 6C, when the objective lens 323 is on a near focal plane, the four-quadrant detector 353 also receives an elliptical light spot. The four-quadrant detector 353 acquires focus signals according to the light intensities on the four quadrants, and drives the objective lens 323 to move longitudinally relative to the carrier disc 40 according to the focus signals (the carrier disc 40 does not move while the objective lens 323 moves longitudinally, or the objective lens 323 does not move while the carrier disc 40 moves longitudinally). In this embodiment, when the objective lens 323 is on the far focal plane, the focusing signal output is greater than 0; when the objective lens 323 is on the near focal plane, the focusing signal output is less than 0; and when the objective lens 323 is on the positive focal plane, the focusing signal output is equal to 0.

The focusing and tracking module 35 is also configured to realize the tracking function. When the four-quadrant detector 353 receives the third detection light L5, according to the luminous flux of the third detection light L5 received by each quadrant (A, B, C, D), the four-quadrant detector 353 can determine whether the first reference light L1 and the second reference light L2 deviate from a preset track (a plurality of tracks, that is, a plurality of annular grooves are formed on the carrier disc 40), and can determine a direction in which the light spot deviates from the track. That is, the four-quadrant detector 353 can obtain tracking signals according to the luminous flux of the third detection light L5 received by each quadrant (A, B, C, D), and drive the carrier disc 40 to move laterally relative to the objective lens 323 according to the tracking signals (the objective lens 323 does not move while the carrier disc 40 moves laterally, or the carrier disc 40 does not move while the objective lens 323 moves laterally).

The dotted line areas in FIG. 7 are the four quadrants A, B, C, and D of the four-quadrant detector 353, and the areas not covered by the ellipse area in the dotted line areas are the areas that can receive the third detection light L5. It can be seen that the more areas covered by the elliptical area in the four quadrants of the four-quadrant detector 353, the less light received and the lower the light intensity. FIG. 8 shows the light intensities obtained by the four quadrants of the four-quadrant detector 353 under the respective situations of t1, t2, t3, t4, and t5 in FIG. 7.

The working process of the test system 10 is described below.

During a first working period of the test system 10, the control module (not shown) outputs control signals to the first laser source 311 and the second laser source 312 to control the first laser source 311 to emit the first reference light L1 while controlling the second laser source 312 to emit the second reference light L2. The first reference light L1 is collimated by the first lens 313, the second reference light L2 is collimated by the second lens 314, and both are incident on the light combining element 321. The light combining element 321 is configured to combine the first reference light L1 and the second reference light and to guide the combined light to the first light splitting element 322. The first light splitting element 322 reflects the received first reference light L1 and second reference light L2 to the objective lens 323. The objective lens 323 focuses the received first reference light L1 and second reference light L2 onto the carrier disc (target sample 20). The fluorescent substance carried by the target sample 20 is excited to generate the first detection light L3 and the second detection light L4 after receiving the first reference light L1 and second reference light L2. The first detection light L3, the second detection light L4, and the reflected first reference light (that is, the detection light L5) are collectively used as the detection light to be received by the objective lens 323. The objective lens 323 guides the first detection light L3, the second detection light L4, and the third detection light L5 to the first light splitting element 322. The first light splitting element 322 transmits the first detection light L3, the second detection light L4, and the third detection light L5 to the second light splitting element 324. The second light splitting element reflects the first detection light L3 and the second detection light L4 to the third light splitting element 325, and transmits the third detection light L5 to the focusing and tracking module 35. The first detection light L3 and the second detection light L4 reflected to the third light splitting element 325 are split by the third light splitting element 325; wherein the first detection light L3 is reflected to the first filter 331, incident to the lens after passing through the first filter 331 to filter out the stray light, and finally enters the first detector 332 after being focused by the lens; the second detection light L4 is transmitted to the second filter 333, incident to the lens after passing through the second filter 331 to filter out the stray light, and finally enters the second detector 334 after being focused by the lens; and the third detection light L5 transmitted to the focusing and tracking module 35 is focused by the lens and finally enters the four-quadrant detector 353 after passing through the astigmatic lens. The control module (not shown) can obtain the base sequence information of the target sample 20 according to the first detection light L3 and the second detection light L4 received by the first detector 332 and the second detector 334. The control module also (not shown) determines whether the first reference light L1 and the second reference light L2 have been focused on the target sample 20 on the carrier disc 40 according to the third detection light L5 received by the four-quadrant detector 353. If there is a deviation, the output positions of the first reference light L1 and the second reference light L2 are adjusted. In a second working period, the first reference light L1 and the second reference light L2 are emitted according to the adjusted positions.

During the above working process, the carrier disc 40 continues to rotate at a high speed, so that the first reference light L3 and the second reference light L4 focus on different positions of the target sample 20, and positions of the first reference light L3 and the second reference light L4 focusing on the carrier disc 40 moves from the center of the carrier disc 40 to the edge (moving from the edge of the carrier disc 40 to the center is also possible).

The above working process is repeated until the detection of the target sample 20 is completed.

The test system 10 provided in this embodiment, on the one hand, replaces the traditional biochip with the carrier disc 40, which is beneficial to reduce the cost of the test system 10 on the basis of maintaining high-speed detection. Because the carrier disc 40 has a small moving space, the overall volume of the test system 10 is small. On the other hand, the test system 10 uses photodiodes (such as avalanche diodes) as the first detector 332 and the second detector 334 to receive the first detection light L3 and the second detection light L4, and can directly perform photoelectric conversion to output electrical signals according to the first detection light L3 and the second detection light L4. Compared with using a camera to take pictures, the photodiode in this embodiment does not need to go through the complex calculation process of image analysis, which is conducive to speeding up data processing, thereby improving the detection speed. In yet another aspect, the test system 10 includes the focusing and tracking module 35, which can effectively improve the accuracy of acquiring biological information of the target sample 20.

The above embodiments are only configured to illustrate but not to limit the technical scheme of the present disclosure, and the present disclosure is described in details only according to the preferable embodiments. Those skilled in the art should understand that they can make the modifications and equivalent replacements according to the technical scheme of the present disclosure without departing from the spirit and scope of technical scheme of the present disclosure, which should be included in the scope of the appended claims of the present disclosure.

## Claims

1. A test system, comprising:
a carrier disc configured to carry a target sample;
a light source module configured for emitting at least two types of reference light with different wavelengths;
a light guiding module located on an emitting path of the reference light and configured for guiding the reference light to the target sample and for receiving and guiding at least two types of detection light with different wavelengths, the detection light being generated by the target sample according to the reference light; and
a detection module configured for receiving the detection light and obtaining biochemical information of the target sample according to the detection light.

2. The test system of claim 1, wherein the light source module comprises a first laser source and a second laser source, the first laser source is configured to emit a first reference light, and the second laser source is configured to emit a second reference light;
the detection light includes a first detection light and a second detection light;
the detection module comprises a first detector for receiving the first detection light and a second detector for receiving the second detection light, the first detector and the second detector are located in different optical paths, the detection module is configured to obtain the biochemical information of the target sample according to the first detection light and the second detection light.

3. The test system of claim 2, wherein the first detector and the second detector are photodiodes.

4. The test system of claim 2, wherein the detection light further comprises part of the first reference light, and the test system further comprises a focusing and tracking module;
The focusing and tracking module is configured to receive the first reference light in the detection light, and to obtain a focus signal and a tracking signal according to the first reference light in the detection light, and the focus signal and the tracking signal are configured to adjust positions where the first reference light and the second reference light are incident on the carrier disc.

5. The test system of claim 4, wherein the light guiding module comprises a first light splitting element, the first light splitting element is configured for guiding the reference light emitted by the light source module to the carrier disc, and for guiding the detection light to the detection module.

6. The test system of claim 5, wherein the light guiding module further comprises a second light splitting element, the second light splitting element is configured to guide the first detection light and the second detection light to the detection module, and to guide the first reference light in the detection light to the focusing and tracking module.

7. The test system of claim 6, wherein the light guiding module further comprises a third light splitting element, the third light splitting element is configured to guide the first detection light to the first detector, and to guide the second detection light to the second detector.

8. The test system of claim 1, wherein the target sample is nucleic acid, the biological information is base sequence information of the target sample.

9. The test system of claim 1, wherein the target sample carries a fluorescent substance, the reference light is laser light, and the detection light is fluorescent light.

10. The test system of any one of claims 1 to 9, further comprising one or more lenses, wherein at least one of the lenses is made of glass.
